# EUROPÄISCHE PATENTSCHRIFT

(11) **EP 1 663 273 B1**
(45) Veröffentlichungstag und Bekanntmachung des Hinweises auf die Patenterteilung: **27.02.2008**
(21) Anmeldenummer: 04763261.7
(22) Anmeldetag: 16.07.2004
(51) Int. Cl.: A61K 36/68, A61K 9/14, A61P 1/10

(54) **PULVERFOERMIGE ZUSAMMENSETZUNG ZUR VERWENDUNG ALS LAXATIVUM**
POWDERY COMPOSITION FOR USE AS A LAXATIVE
COMPOSITION PULVERULENTE SERVANT DE LAXATIF

(30) Priorität: 05.09.2003 DE 10341403; 04.10.2003 DE 10346083
(43) Veröffentlichungstag der Anmeldung: 07.06.2006
(73) Patentinhaber: Madaus GmbH, 51067 Köln (DE)
(72) Erfinder: SCHATA, Martin, 40489 Düsseldorf (DE); PULLEN, Christian, 51061 Köln (DE)
(74) Vertreter: Gesthuysen, Michael
(86) Internationale Anmeldenummer: PCT/EP2004/007905
(87) Internationale Veröffentlichungsnummer: WO 2005/027948

(56) Entgegenhaltungen:
- BE-A- 887 111
- GB-A- 2 067 402
- US-A- 4 511 561
- US-A- 5 167 959
- US-A- 5 834 026
- US-B2- 6 576 253

## Beschreibung

Die vorliegende Erfindung betrifft eine pulverförmige Zusammensetzung zur Verwendung als Laxativum (Abführmittel, Laxans). Insbesondere betrifft die vorliegende Erfindung eine pulverförmige Zusammensetzung von Plantagosamen und/oder Plantagosamenschalen sowie anthranoiden Verbindungen, insbesondere Sennosiden, vorzugsweise in Form von anthranoidhaltigen, insbesondere sennosidhaltigen Pflanzenteilen oder Pflanzenbestandteilen, sowie die Verwendung dieser Zusammensetzung als Laxativum (Abführmittel, Laxans).

Laxativa - synonym auch als "Laxantien" oder "Abführmittel" bezeichnet - gibt es in vielfältigsten Erscheinungsformen, hierunter auch zahlreiche pflanzliche Abführmittel.

Die BE 887 111 A offenbart ein Laxativum aus Plantagosamen und Sennesfrüchten. Des weiteren kann diese Zubereitung auch Gummi arabicum oder Paraffin enthalten.

Die US 4 511 561 A offenbart ein Abführmittel auf Basis von Psylliumsamen und Sennafrüchten. Die spezielle Zusammensetzung dieser Zubereitung enthält zudem Tragacanthmasse, Gummi arabicum, Paraffine sowie pflanzliche Öle verschiedener Herkunft.

Die US 5 167 959 A umfaßt laxative Zusammensetzungen pflanzlicher Herkunft von weichmachendem und schleimbildendem Charakter aus gehärteten "Mikrobedlets", welche Flüssigparaffin sowie weitere inerte Zusatzstoffe enthalten. Insbesondere wird auf einen pulverförmigen Extrakt aus Plantago und Guargummi als abführende Komponenten hingewiesen.

Die US 5 834 026 A betrifft eine diätetische Zubereitung mit Polysacchariden pflanzlicher Herkunft aus Psyllium, Cellulose sowie Guargummi.

Die GB 2 067 402 A offenbart ein Abführmittel mit Sennesfrüchten sowie Psylliumsamen, wobei die genaue Zusammensetzung auch Zusätze wie Gummi arabicum, Talkum, Eisenoxid, Tragacanthgummi sowie Hartparaffin und Saccharum beinhaltet.

Die US 6 576 253 A offenbart vitaminhaltige Nahrungsriegel für schwangere Frauen, wobei diese Riegel auch Wirkstoffe gegen Verstopfung enthalten, da während der Schwangerschaft und Stillzeit die Darmtätigkeit beeinträchtigt werden kann.

Weiterhin ist beispielsweise die Verwendung von Plantagosamen (Plantaginis ovatae Semen bzw. Semen plantaginis ovatae, synonym auch als Indischer Flohsamen, Indisches Psyllium, Blondes Psyllium, Ispaghula oder Semen Ispaghulae bezeichnet) oder seiner Samenschalen (Plantagosamenschalen, Plantaginis ovatae seminis integumentum) für Arzneimittel zur Regulierung der Darmtätigkeit bekannt. Plantagosamen besitzt eine beträchtliche Quellfähigkeit und übt einen physikalischen Dehnungsreiz auf die empfindlichen Rezeptoren der Darmwände aus. Nach einem bekannten Verfahren (vgl. DE-PS 11 03 520) werden die Samen fein vermählen, mit Wasser zu einem viskosen Brei angeteigt und in Strangform getrocknet, zerkleinert und schließlich dragiert.

Die Wirkung der Sennespflanze (Sennapflanze, Cassia senna L. und Cassia angustifolia Vahl), insbesondere ihrer Früchte (Fructus Sennae, synonym auch als Sennesschoten oder Sennesbälge bezeichnet) (z. B. Alexandriner-Sennesfrüchte = Sennae fructus acutifoliae und/oder Tinnevelly-Sennesfrüchte = Sennae fructus angustifoliae) bzw. ihrer Fruchthülsen wie auch ihrer Fiederblätter (Folia Sennae), als pflanzliches Abführmittel ist ebenfalls bekannt.

Darüber hinaus kennt man auch Laxativa, welche die beiden vorgenannten Wirkungsprinzipien vereinen, beispielsweise Laxativa, bei denen die physikalische Wirkung der Plantagosamen durch das pharmakologisch anregende Wirkprinzip der Sennoside, der Inhaltsstoffe der Sennesfrüchte, unterstützt wird, um eine bessere Gesamtwirkung zu erreichen.

So ist es bereits bekannt, Mischungen aus vermahlenem Plantagosamen und Sennesfrüchten als einfaches Gemisch, bei dem die Bestandteile nebeneinander vorliegen, zu konfektionieren. Jedoch ist bei derartigen Gemischen die Rieselfähigkeit und Suspendierbarkeit in Wasser nicht optimal einzustellen, was für die orale Applikation aber wünschenswert wäre. Auch ist die Verarbeitung dieser Gemische zu applikationsfertigen Pulvern nicht oder nicht ohne weiteres möglich.

Um den vorgenannten Nachteilen abzuhelfen, ist in der DE 30 01 357 C2 ein Laxativum in Form eines Abführmittelgranulates auf der Basis von Sennesfrüchten, Plantagosamen und gegebenenfalls Plantagosamenschalen mit erhöhter Retardwirkung vorgeschlagen worden, bei denen die Sennapartikel von Plantagosamen, insbesondere Schleimstoffen der Plantogasamen, umschlossen bzw. umhüllt vorliegen. Die dort beschriebene Zusammensetzung ist ein effizientes Laxativum. Da die Zusammensetzung aber als Granulat mit etwa 1 bis etwa 3 mm Korngröße der Granulatteilchen vorliegt, d. h. als asymmetrische Aggregate von Pulverpartikeln, vorliegt, muß sie mit reichlich Wasser eingenommen werden. Ansonsten besteht die Gefahr, daß es bei unsachgemäßer oraler Einnahme bzw. Applikation, also bei der oralen Einnahme bzw. Applikation mit zu geringen Wassermengen, zur Bildung von aufgequollenen Klumpen des Granulats kommen kann, die in den schlimmsten Fällen zu einem Verschluß der Speiseröhre oder aber sogar des Magen-Darm-Traktes führen können.

Auf dem Markt befindliche Laxativa auf Basis von Flohsamen oder indischem Flohsamen (Psyllium) oder deren Bestandteilen liefern oftmals keine ausreichende abführende Wirkung und bleiben nach Suspension in Wasser aufgrund der rasch einsetzenden Quellwirkung des Flohsamens nicht lange trinkfähig, so daß sie unmittelbar nach Herstellung der wäßrigen Suspension eingenommen werden müssen. Denn nach Suspension in Wasser gelieren diese Produkte bereits nach wenigen Minuten und bilden einen dicken, untrinkbaren Klumpen auf der Oberfläche. Zusätzlich erfolgt bei den meisten Präparaten sehr schnell mindestens eine zweischichtige Phasentrennung, welche die homogene Verteilung der wirksamen Komponenten in dem angerührten Präparat nicht mehr gewährleistet. Auch diese Präparate müssen mit reichlich Wasser eingenommen werden, weil ansonsten die Gefahr besteht, daß es zur Bildung von aufgequollenen Klumpen kommen kann, die in den schlimmsten Fällen zu einem Verschluß der Speiseröhre oder aber sogar des Magen-Darm-Traktes führen können. Weiterhin ist bei diesen Produkten auch der fade Geschmack und das künstliche Aussehen nachteilig.

Somit liegt der vorliegenden Erfindung die Aufgabe zugrunde, eine Zusammensetzung zur Verwendung als Laxativum bereitzustellen, welches die vorgeschilderten Nachteile zumindest weitgehend vermeidet.

Eine weitere Aufgabe der vorliegenden Erfindung ist die Bereitstellung einer als Laxativum verwendbaren Zusammensetzung, die einerseits eine gute abführende Wirkung zeigt, aber gleichzeitig auch eine erleichterte, komplikationsfreie Applikation ermöglicht, insbesondere ohne die vorgeschilderten Probleme bzw. Risiken.

Schließlich ist eine weitere Aufgabe der vorliegenden Erfindung die Weiterentwicklung des in der DE 30 01 357 C2 beschriebenen Laxativums.

Die Anmelderin hat nun überraschenderweise herausgefunden, daß sich eine Zusammensetzung bzw. Mischung auf Basis von Plantagosamen und/oder Plantagosamenschalen, sowie anthranoiden Verbindungen mit laxativer bzw. abführender Wirkung, insbesondere Sennosiden, vorzugsweise in Form von anthranoidhaltigen bzw. sennosidhaltigen Pflanzen(bestand)teilen (z. B. Früchten bzw. Fiederblättern von Sennespflanzen), zu einem feinteiligen Pulver verarbeiten läßt, welches die vorgenannten Nachteile bei seiner Verwendung als Laxativum vermeidet, wenn man diese Zusammensetzung zusammen mit einem Polygalactomannan oder dessen Derivat formuliert.

Gegenstand der vorliegenden Erfindung ist somit eine pulverförmige Zusammensetzung, welche
(A) Plantagosamen (Plantaginis ovatae Semen) und/oder (A') Plantagosamenschalen; und
(B) mindestens eine anthranoide Verbindung, insbesondere mindestens ein Sennosid, vorzugsweise in Form von anthranoidhaltigen, insbesondere sennosidhaltigen Pflanzenteilen oder Pflanzenbestandteilen,
enthält, wobei die pulverförmige Zusammensetzung außerdem (C) mindestens ein Polygalactomannan oder dessen Derivat enthält.

Da die mindestens eine anthranoide Verbindung, insbesondere das Sennosid, vorzugsweise in Form eines anthranoid- bzw. sennosidhaltigen Pflanzenteils bzw. Pflanzenbestandteils (z. B. Früchte bzw. Fruchthülsen/-schoten oder Fiederblätter von Sennespflanzen) zugesetzt wird, liegt im allgemeinen eine komplexe Mischung verschiedener anthranoider Verbindungen bzw. Sennoside in der erfindungsgemäßen Zusammensetzung vor.

Denn die Anmelderin hat überraschenderweise herausgefunden, daß das Polygalactomannan oder dessen Derivat zum einen als (Co-)Stabilisator in bezug auf die zuvor definierte pulverförmige Zusammensetzung fungiert, wenn diese in wäßrige Suspension gebracht wird; aufgrund der Anwesenheit des polygalactomannanbasierten oder -derivatisierten Polysaccharids ist also die pulverförmige Zusammensetzung nach der vorliegenden Erfindung verbessert in Wasser suspendierbar bzw. dispergierbar und bleibt eine wäßrige Suspension der erfindungsgemäßen pulverförmigen Zusammensetzung über einen längeren Zeitraum stabil und somit trinkbar, d. h. die Suspension geliert nicht vorzeitig und führt auch zu keiner vorzeitigen Phasentrennung. Darüber hinaus hat das Polygalactomannan aber auch in unerwarteter Weise die Funktion, zusätzlich auch die Wirkung der übrigen Bestandteile, insbesondere der Plantagosamen und gegebenenfalls der Plantagosamenschalen, zu unterstützen, weil es selbst eine gewisse Quellfähigkeit aufweist; es wirkt also in unerwarteter Weise synergistisch mit den anderen Bestandteilen der pulverförmigen Zusammensetzung zusammen und steigert auf diese Weise deren pharmakologische Wirksamkeit. Schließlich unterstützt das Polygalactomannan oder dessen Derivat überraschenderweise auch die Verarbeitbarkeit der erfindungsgemäßen Zusammensetzung zu einem feinteiligen, rieselfähigen Pulver, welches in Wasser suspendierbar bzw. dispergierbar ist.

Vorteilhafterweise wird das Polygalactomannan oder sein Derivat in Form von Verbindungen aus der Gruppe von Guaran (synonym auch als Guar-Gummi oder Cyamopsis-Gummi bezeichnet) oder Guar-Derivaten, insbesondere partiell oder vollständig veresterten und/oder veretherten Guar-Derivaten, insbesondere Guar-Ethern, zugesetzt, wie zum Beispiel Carboxymethyl- und Hydroxyalkyl-Derivaten und kationisch modifizierten Produkten aus der Umsetzung von Guar-Mehl mit Monochloressigsäure, Ethylen- oder Propylenoxid und 2,3-Epoxypropyltrimethylammoniumchlorid in Gegenwart von Alkali.

Besonders bevorzugt wird das Polygalactomannan oder sein Derivat in Form von Guar-Mehl und/oder Guar-Gummi, vorzugsweise Guar-Gummi, zugesetzt. Das Guar-Mehl und/oder das Guar-Gummi wirkt nicht nur in besonderer Weise als (Co-)Stabilisator der erfindungsgemäßen Zusammensetzung in wäßriger Suspension, sondern es unterstützt auch die Formulierung eines besonders feinteiligen Pulvers und steigert aufgrund seiner eigenen Quellfähigkeit in besonderer Weise auch die abführende Wirkung der übrigen Bestandteile, wirkt also mit den übrigen Bestandteilen in synergistischer Weise zusammen.

Guar-Mehl ist die Bezeichnung für ein grau-weißes Pulver, welches durch Mahlen des Endosperms der ursprünglich im indischen und pakistanischen Raum endemischen, inzwischen auch in anderen Ländern, zum Beispiel im Süden der USA, kultivierten, zur Familie der Leguminosen gehörenden Guar-Bohne (Cyamopsis tetragonoloba) gewonnen wird. Hauptbestandteil des Guar-Mehls ist mit bis zu ca. 85 Gew.-% der Trockensubstanz das Guaran (Guar-Gummi, Cyamopsis-Gummi); Nebenbestandteile sind Proteine, Lipide und Cellulose. Guaran selbst ist ein Polygalactomannan, d. h. ein Polysaccharid, dessen lineare Kette aus nichtsubstituierten und in der C₆-Position mit einem Galactose-Rest substituierten Mannose-Einheiten in β-D-(1→4)-Verknüpfung aufgebaut ist. Guaran selbst besitzt eine extrem hohe Verdickungswirkung (Viskosität einer 1,5 gew.%igen wäßrigen Lösung bis 15.000 mPa • s). Die Lösungen sind durch unlösliche Nebenbestandteile des Guar-Mehls aber stark getrübt. Die Löslichkeit kann durch die Derivatisierung, insbesondere durch Veretherung oder Veresterung des Guar-Mehls, erheblich verbessert werden. Für weitere Einzelheiten kann verwiesen werden auf Römpp Chemielexikon, 10. Auflage, Band 2, 1997, Seiten 1622/1623, Stichworte "Guar-Mehl" und "Guar-Derivate", Georg Thieme Verlag Stuttgart/New York, und die dort genannten Literaturstellen, deren jeweiliger Inhalt durch Bezugnahme eingeschlossen ist.

Unter Guar-Derivaten versteht man erfindungsgemäß insbesondere Derivate des Guar-Mehls. Guaran, der Hauptbestandteil des Guar-Mehls, läßt sich als Polysaccharid auf unterschiedlichen Wegen, zum Beispiel durch vollständige oder partielle Veresterung und/oder Veretherung seiner Hydroxy-Gruppen, derivatisieren. Technische Bedeutung erlangt haben insbesondere die Guar-Ether, insbesondere die Carboxymethyl- und Hydroxyalkyl-Derivate sowie kationisch modifizierte Produkte, die bei der Umsetzung von Guar-Mehl mit Monochloressigsäure, Ethylen- oder Propylenoxid und 2,3-Epoxypropyltrimethylammoniumchlorid in Gegenwart von Alkali anfallen. Die Guar-Derivate, insbesondere Guar-Ether, zeichnen sich gegenüber dem nichtmodifizierten Guar-Mehl durch eine schnellere und bessere Löslichkeit in Wasser und eine erhöhte Transparenz der wäßrigen Lösung aus.

Besonders vorteilhaft ist es, wenn die erfindungsgemäße pulverförmige Zusammensetzung außerdem (D) mindestens eine Kieselsäure oder deren Derivat enthält. Die Kieselsäure unterstützt einerseits die Rieselfähigkeit der pulverförmigen Zusammensetzung und erleichtert deren Verarbeitbarkeit zu einem feinteiligen Pulver, d. h. wirkt sich positiv bzw. beschleunigend auf den Produktionsprozeß aus, indem es als Fließvermittler wirkt. Andererseits wirkt die Kieselsäure (co-)stabilisierend in einer wäßrigen Suspension der erfindungsgemäßen Zusammensetzung und unterstützt somit die Wirkung des Polygalactomannans oder seines Derivats, insbesondere durch synergistische Interaktion mit dem Polygalactomannan oder seinem Derivat. Schließlich adsorbiert die Kieselsäure bei der Verarbeitung oder Lagerung gegebenenfalls auftretende Feuchtigkeit und wirkt auf diese Weise als ein die Verklumpung verhinderndes Mittel.

Als Kieselsäure besonders bewährt hat sich eine hochdisperse, vorzugsweise pyrogene Kieselsäure bevorzugt mit einem SiO₂-Gehalt von 95 % und mehr, insbesondere von 99 % und mehr. Ein Beispiel für eine erfindungsgemäß geeignete Kieselsäure ist das Produkt AEROSIL^{®} der Fa. Degussa; hierbei handelt es sich um eine durch Hydrolyse von Siliciumtetrachlorid in einer Knallgasflamme (2 H₂ + O₂ + SiCl₄ → SiO₂ + 4 HCl, Flammenhydrolyse) hergestellte, hochdisperse pyrogene Kieselsäure von über 99,8 % SiO₂-Gehalt. Ein Teil der Kieselsäure bzw. ihres Derivates kann durch Maltodextrin ersetzt sein.

Was das oder die erfindungsgemäß eingesetzten Sennoside anbelangt, so handelt es sich hierbei im allgemeinen um Hydroxyanthracenderivate, insbesondere 1,8-Dihydroxyanthronderivate. Im allgemeinen wird ein komplexes Gemisch verschiedener Sennoside zum Einsatz gebracht.

Insbesondere ist das erfindungsgemäß eingesetzte Sennosid ausgewählt aus der Gruppe der folgenden Verbindungen der allgemeinen Formel (I): 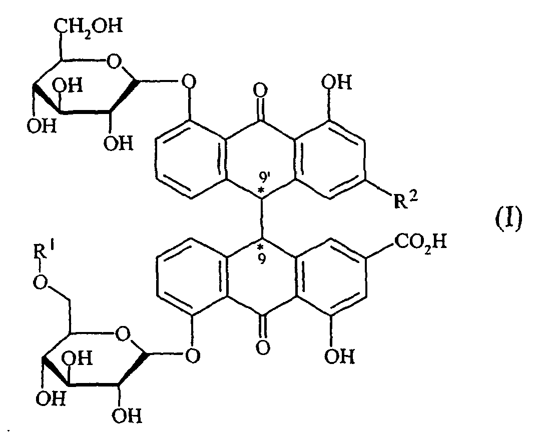 wobei in der allgemeinen Formel (I)
- der Rest R¹ Wasserstoff oder eine Gruppe -CO-CO₂H darstellt,
- der Rest R² eine Gruppe -CO₂H oder -CH₂OH darstellt, jedoch mit der Maßgabe, daß, wenn R¹ eine Gruppe -CO-CO₂H bezeichnet, R² eine Gruppe -CO₂H darstellt,
- die mit dem Zeichen "*" gekennzeichneten Kohlenstoffatome in 9- und 9'-Position des Anthrongerüstes Chiralitätszentren darstellen,
sowie Mischungen und/oder Stereoisomeren, insbesondere Enantiomeren und/ oder Diastereoisomeren, und/oder Derivaten der vorgenannten Verbindungen.

Vorzugsweise ist das Sennosid ausgewählt aus der Gruppe der folgenden Verbindungen der allgemeinen Formel (I):

| Verbindung | R¹ | R² | 9-9' |
|---|---|---|---|
| (I A) | -H | -CO₂H | R*,R* (threo) |
| (I B) | -H | -CO₂H | R*,S* (erythro) |
| (I C) | -H | -CH₂OH | R*,R* (threo) |
| (I D) | -H | -CH₂OH | R*,S* (erythro) |
| (I E) | -CO-CO₂H | -CO₂H | R*,R* (threo) |
| (I F) | -CO-CO₂H | -CO₂H | R*,S* (erythro) |

sowie Mischungen und/oder Derivaten der vorgenannten Verbindungen.

Vorteilhafterweise werden die Sennoside in Form von Früchten (Fructus), insbesondere z. B. Fruchthülsen, und/oder Fiederblättern (Folia), vorzugsweise Früchten oder Fruchthülsen, von Sennapflanzen zugesetzt. Bevorzugt sind die Tinnevelly-Sennapflanze und die Alexandriner-Sennapflanze, besonders bevorzugst die Tinnevelly-Sennapflanze, vorzugsweise in getrockneter und zu feinem Pulver vermahlener Form.

Ganz besonders bevorzugt setzt man die Sennoside in Form von Früchten (Fructus) (insbesondere z. B. Fruchthülsen/-schoten) und/oder Fiederblättern (Foliae), vorzugsweise Früchten, der Tinnevelly-Sennapflanze (Senna angustifolia, Cassia angustifolia) ein.

Die Tinnevelly-Sennapflanze (Senna angustifolia, Cassia angustifolia) ist ein bis zu zwei Meter hoher Strauch, der in den an das Rote Meer grenzenden Ländern beheimatet ist. Er wird jedoch in größerem Ausmaße in Indien in Feldkulturen, häufig im Wechsel mit Reis angebaut. Dieser Strauch wird nicht nur im Umkreis der Stadt Tinnevelly (Drogenbezeichnung), sondern auch in der Nähe von Bombay und Madras angebaut. Die Alexandriner-Sennapflanze ist eine in Nordafrika und im mittleren Nilgebiet heimischer, bis zu 60 cm hoch werdender Halbstrauch, der in Ägypten und im Sudan angebaut wird. Seinen Namen verdankt er dem früheren Ausfuhrhafen Alexandria, und wird deshalb auch als Khartum-Senna bezeichnet. Früchte und Fiederblätter der Sennapflanzen enthalten ein komplexes Sennosidgemisch, welches abführende Wirkung hat. Für weitere Einzelheiten kann verwiesen werden auf Deutsches Arzneibuch, 10. Ausgabe (DAB 10), Deutscher Apotheker Verlag Stuttgart, 1991, und den Kommentar zum DAB 10. Ausgabe, 1. Lfg., 1993, T 54, Seiten 1 bis 5, auf die Dissertationsschrift der Gerhard-Mercator-Universität Duisburg "NIR-Spektrometrie als Methode der quantitativen Analyse von synthetischen und pflanzlichen Wirkstoffen in Tabletten und Granulaten" des Herrn Dr. Frank Zeyen aus dem Jahre 2000 sowie auf die WHO-Monographie (WHO Monographs on Selected Medicinal Plants) "Folium Sennae" (*http:*//*www.who.int*/*medicines*/*library*/*trm*/*medicinalplants*/*pdf*/*241to249.pdf*)

Als anthranoidhaltige Pflanzen(bestand)teile können erfindungsgemäß aber auch solche von anderen anthrachinonhaltigen Drogen mit laxativer Wirkung eingesetzt werden, z. B. Rheum palmatum und Rheum officinale (Medizinal-Rhabarber, Wurzel), Rhamnus frangula (Faulbaum, Rinde), Rhamnus purshiana (amerikanischer Faulbaum, Rinde), Aloe barbadensis, Aloe ferox (Curaçao- bzw. Kap-Aloe, eingedickter Saft aus Blättern), Rhamnus catharicus (Kreuzdorn, Beeren) etc., um nur einige zu nennen.

Gemäß einer besonderen Ausführungsform der vorliegenden Erfindung liegen die Anthranoide bzw. Sennoside, insbesondere die Sennapflanzenpartikel (Früchte bzw. Fruchthülsen und/oder Fiederblätter), in einer von den Plantagosamen, insbesondere von den Schleimstoffen der Plantagosamen, zumindest teilweise oder vollständigen Umhüllung in der erfindungsgemäßen Zusammensetzung vor. Auf diese Weise wird die erfindungsgemäße Zusammensetzung zusätzlich stabilisiert.

Die Mengenanteile der einzelnen Bestandteile sind nicht kritisch und kann in weiten Bereichen variieren. Beispielsweise kann der Gehalt an Plantagosamen im Bereich von 30 bis 80 Gew.-%, insbesondere 40 bis 70 Gew.-%, vorzugsweise 45 bis 55 Gew.-%, variieren. Der Gehalt an gegebenenfalls vorhandenen Plantagosamenschalen kann ebenfalls in weiten Bereichen variieren, nämlich im Bereich von 0 bis 5 Gew.-%, insbesondere 1 bis 4 Gew.-%, vorzugsweise 1,5 bis 3 Gew.-%. Der Gehalt an Sennosid(en) kann ebenfalls in weiten Bereichen variieren; im allgemeinen beträgt er 0,05 bis 1,0 Gew.-%, insbesondere 0,08 bis 0,6 Gew.-%, vorzugsweise 0,1 bis 0,5 Gew.-%. Der Gehalt an Sennapflanze(n), insbesondere Sennafrüchten oder Sennafruchthülsen, kann im Bereich von 5 bis 25 Gew.-%, insbesondere 7,5 bis 15 Gew.-%, vorzugsweise 10 bis 13 Gew.-%, variieren. Der Gehalt an Polygalactomannan(en), insbesondere Guaran und/oder Guar-Derivaten (insbesondere Guar-Mehl oder Guar-Gummi), kann im Bereich von 5 bis 20 Gew.-%, insbesondere 7 bis 15 Gew.-%, vorzugsweise 7,5 bis 12 Gew.-%, besonders bevorzugt 8,0 bis 10 Gew.-%, variieren. Der Gehalt an gegebenenfalls vorhandener Kieselsäure oder ihren Derivaten kann im Bereich von 0 bis 5 Gew.-%, insbesondere 0,01 bis 2 Gew.-%, vorzugsweise 0,02 bis 1 Gew.-%, besonders bevorzugt 0,05 bis 0,1 Gew.-%, variieren. Alle vorgenannten Gewichtsprozentangaben sind jeweils auf das Trockengewicht der erfindungsgemäßen Gesamtzusammensetzung bezogen.

Zur Verbesserung der Verarbeitbarkeit und/oder des Aussehens und/oder der organoleptischen Eigenschaften können der erfindungsgemäßen Zusammensetzung außerdem weitere Additive bzw. Zusatzstoffe zugesetzt sein, beispielsweise Farbstoffe (z. B. natürliche oder naturidentische Farbstoffe), Geschmacksmittel, Geschmacksverstärker, Aromastoffe, (Co-)-Stabilisatoren, Füllstoffe, Verarbeitungshilfsmittel, Süßungsmitteln etc. sowie Mischungen der vorgenannten Verbindungen.

Eine erfindungsgemäß bevorzugte pulverförmige Zusammensetzung hat die folgende Formulierung, wobei die Mengenangaben jeweils auf das Trockengewicht der Gesamtzusammensetzung bezogen sind:

| | Gewichtsteile |
|---|---|
| • (A) Plantagosamen: | 30 bis 80, insbesondere 40 bis 70, vorzugsweise 45 bis 55 |
| • (A') Plantagosamenschalen: | 0 bis 5, insbesondere 1 bis 4, vorzugsweise 1,5 bis 3 |
| • (B) Tinnevelly-Senilafrüchte: | 5 bis 25, insbesondere 7,5 bis 15, vorzugsweise 10 bis 13 (entsprechend an Sennosiden 0,05 bis 1,0, insbesondere 0,08 bis 0,6, vorzugsweise 0,1 bis 0,5 Gewichtsteile) |
| • (C) Guar-Mehl und/oder Guar-Gummi: | 5 bis 20, insbesondere 7 bis 15, vorzugsweise 7,5 bis 12, besonders bevorzugt 8,0 bis 10 |
| • (D) Kieselsäure: | 0 bis 5, insbesondere 0,01 bis 2, vorzugsweise 0,02 bis 1, besonders bevorzugt 0,05 bis 1 |
| • (E) Geschmacks- und Farbstoffe: | 0 bis 60, insbesondere 20 bis 50, vorzugsweise 35 bis 45 |

Die Verarbeitbarkeit der einzelnen Wirkbestandteile zu einer stabilen, feinteiligen, homogenen Pulvermischung wird Studien der Anmelderin zufolge erst durch die Einarbeitung des erfindungsgemäß eingesetzten Polygalactomannans oder seines Derivates, insbesondere Guar-Mehl und/oder Guar-Gummi, gegebenenfalls zusammen mit einer feinteiligen Kieselsäure, möglich, wobei das Polygalactomannan bzw. sein Derivat, gegebenenfalls zusammen mit einer feinteiligen Kieselsäure, zusätzlich stabilisierend in bezug auf eine wäßrige Suspension der erfindungsgemäßen Pulverzusammensetzung wirkt, was die orale Einnahme deutlich erleichtert.

Der Hauptmassenanteil der erfindungsgemäßen Zusammensetzung, insbesondere mehr als 60 Gew.-%, insbesondere mehr als 70 Gew.-%, vorzugsweise mehr als 75 Gew.-%, weist Korngrößen im Bereich von 125 bis 250 µm auf.

Im allgemeinen weist die erfindungsgemäße Zusammensetzung die folgende Korngrößenverteilung auf (Siebanalyse nach DIN ISO 3310-1):
- mehr als 95 Gew.-%, insbesondere mehr als 99 Gew.-%, feiner als 500 µm;
- mehr als 70 Gew.-%, insbesondere mehr als 75 Gew.-%, feiner als 250 µm ;
- mehr als 10 Gew.-%, insbesondere mehr als 15 Gew.-%, feiner als 125 µm.

In vorteilhafter Weise liegen die einzelnen Bestandteile der erfindungsgemäßen Zusammensetzung in einer innigen, vorzugsweise homogenen Mischung vor, d. h. die einzelnen Bestandteile sind innig, vorzugsweise homogen, miteinander verarbeitet bzw. vermengt.

Die erfindungsgemäße Zusammensetzung ist außerordentlich quellfähig. Dadurch wird eine Anregung der Darmtätigkeit über physikalische Reize erreicht. Die Quellungszahl (nach DAB 8, Deutsches Arzneibuch, 8. Ausgabe 1978, amtliche Ausgabe, Deutscher Apotheker Verlag Stuttgart/Govi-Verlag GmbH Frankfurt, Seiten 24 ff.) beträgt insbesondere mindestens 5,0, insbesondere mindestens 6,0, vorzugsweise mindestens 7,0, besonders bevorzugt mindestens 7,5, ganz besonders bevorzugt mindestens 8,0 und mehr.

Wie zuvor beschrieben, können dabei die Partikel der vermahlenen Sennafrüchte gegebenenfalls von einer Schutzschicht aus den Schleimstoffen der Plantagosamen-Mahlprodukte umgeben sein. Durch diese Umhüllung werden die Sennoside weitgehend vor unerwünschten Veränderungen geschützt und ihre Freigabe verzögert, so daß eine noch länger anhaltende, retardierende und mildere Wirkung erzielt werden kann.

Versuche, welche die Anmelderin mit der erfindungsgemäßen Zusammensetzung durchgeführt hat, belegen eine verzögerte Freisetzung der Sennoside aus dem erfindungsgemäßen Laxativum, wobei die retardierte Freigabe der Sennoside jedoch keineswegs zu einer völligen Blockierung der Wirkstoffe führt, sondern vielmehr zu einer gewünschten, über längere Zeit hinweg praktisch vollständigen Freisetzung. Somit können die Sennoside vollständig zur Auswirkung kommen.

Die erfindungsgemäße pulverförmige Zusammensetzung eignet sich somit in ausgezeichneter Weise als Laxativum (Abrührmittel).

Aufgrund der Tatsache, daß die erfindungsgemäße Zusammensetzung in Form eines feinteiligen Pulvers vorliegt, läßt sie sich für die orale Applikation besonders gut in Wasser einarbeiten bzw. dispergieren und bleibt auch lange Zeit nach dem Einrühren in Wasser sehr gut trinkbar, d. h. im Gegensatz zu den Produkten des Standes der Technik findet keine rasche Phasentrennung statt. Im Vergleich dazu gelieren Produkte des Standes der Technik bereits nach wenigen Minuten und bilden einen dicken, untrinkbaren Klumpen auf der Oberfläche.

Darüber hinaus besitzt die erfindungsgemäße Zusammensetzung ein naturgetreues Aussehen und einen naturgetreuen Geschmack.

Die erfindungsgemäße Zusammensetzung zeigt eine gute abführende Wirkung und zeichnet sich in Folge einer sanften und bei bestimmungsgemä-ßer Anwendung von unerfreulichen Nebeneffekten weitestgehend freien Wirkung besonders durch eine gute Verträglichkeit aus. Somit eignet sie sich zur Unterstützung der Darmtätigkeit insbesondere auch bei Hämorrhoid- oder Fissur-Patienten und selbst nach einem chirurgischen Eingriff. Gleichermaßen kann die erfindungsgemäße Zusammensetzung zur klinischen Regulierung für immobilisierte Patienten, d. h. insbesondere Patienten, die ans Bett gefesselt sind, über längere Zeit hinweg angewandt werden und ist auch ohne Bedenken während der Schwangerschaft verabreichbar. Die erfindungsgemäße Zusammensetzung kann somit bei allen Erkrankungen zum Einsatz kommen, bei denen eine leichte Defäkation mit weichem Stuhl erwünscht ist (z. B. bei Analfissuren, bei Hämorrhoiden, nach rektal-analen operativen Eingriffen, zur Reinigung des Darms vor Röntgenuntersuchungen sowie vor und nach operativen Eingriffen des Bauchraums bzw. im Gastrointestinaltrakt, bei Obstipationen etc.).

Beispielsweise ist die erfindungsgemäße Zusammensetzung ebenfalls besonders geeignet für die Behandlung von Patienten mit medikamentös bedingter Obstipation, z. B. bei der Behandlung mit dem häufig angewendeten Loperamid^{®}. Eine Studie von Ewe et al. (Pharmacology 1993, 47, Suppl. 1, Seiten 242 bis 248) belegt die verkürzte Darmpassage bei gleichzeitiger Anwendung von Loperamid^{®} und sennosidhaltigen Präparaten. Es wurde nun überraschend gefunden, daß unter anderem aufgrund der "flüssigen" Darreichung der erfindungsgemäßen Zusammensetzung in Form einer wäßrigen Suspension und der feinteiligen Partikel dieser Effekt besser als bei den herkömmlichen Präparaten ist, da die flüssige Anwendung in Form einer wäßrigen Suspension feinster Partikel auch bei vorübergehender Einschränkung der Passage vom Magen in den Darm für feste feinteilige Partikel im Rahmen der "flüssigen" Zubereitung (wäßrige Suspension) noch möglich ist. Folglich eignet sich die erfindungsgemäße Zusammensetzung auch zur Erleichterung der Darmpassage und Defäkation, insbesondere als Linderung der Nebenwirkungen, bei Therapie mit Medikamenten, die - wenn auch nur als unerwünschte Nebenwirkungen - Obstipation verursachen.

Toxizitätsstudien belegen, daß die erfindungsgemäße Zusammensetzung weder eine akute noch bei bestimmungsgemäßer Anwendung eine chronische Toxizitätsgefahr mit sich bringt. Es wurde auch keinerlei choleritische Wirkung beobachtet. Es wurden auch keinerlei Unverträglichkeiten festgestellt.

Somit eignet sich die erfindungsgemäße pulverförmige Zusammensetzung in ausgezeichneter Weise als effizientes Laxativum, welches bei der bestimmungsgemäßen oralen Einnahme zu keinen unerwünschten Nebeneffekten, wie insbesondere Verstopfung der Speiseröhre oder des Magen-Darm-Traktes, führt.

Die erfindungsgemäße Zusammensetzung vereinigt somit in effizienter Weise die physikalische Wirkung der Plantagosamen aufgrund von Quelleigenschaften und das pharmakologisch anregende Wirkprinzip der Inhaltsstoffe der Sennafrüchte, der Sennoside, um auf diese Weise eine verbesserte Gesamtwirkung zu erreichen.

Aufgrund ihres pulverförmigen Charakters wird die erfindungsgemäße Zusammensetzung zur oralen Anwendung in Wasser in Suspension gebracht. Die leichte Suspendierbarkeit und nachfolgende Stabilisierung der so hergestellten Suspension ist durch das polygalactomannanbasierte oder -derivatisierte Polysaccharid, insbesondere in Form von Guar-Mehl und/oder Guar-Crum, gegebenenfalls zusammen mit der Kieselsäure, gewährleistet, so daß die orale Einnahme automatisch mit ausreichenden Wassermengen erfolgt, da sie als wäßrige Suspension zugeführt wird.

Vorteilhafterweise liegt die erfindungsgemäße Zusammensetzung zu diesem Zweck bereits in Portionsbeuteln verpackt vor, deren jeweils enthaltene Menge an erfindungsgemäßer Zusammensetzung vorzugsweise einer Einzeldosis entspricht (z. B. jeweils 5 bis 10 g der erfindungsgemäßen Zusammensetzung oder z. B. solche Mengen an Zusammensetzung, die 5 bis 20 mg Sennosiden entsprechen). Um die erfindungsgemäße Zusammensetzung ausreichend zu schützen, sind die Portionsbeutel vorzugsweise zumindest im wesentlichen luftdicht und/oder zumindest im wesentlichen wasserdicht ausgebildet (z. B. durch Verschweißen oder Verkleben). Vorteilhafterweise schützen die Portionsbeutel die erfindungsgemäße Zusammensetzung auch vor Lichteinwirkung, insbesondere UV-Licht.

Die erfindungsgemäße Zusammensetzung wird im allgemeinen oral eingenommen, insbesondere in Mengen von 1 bis 50 g, insbesondere 1 bis 20 g, vorzugsweise 5 bis 10 g der Zusammensetzung per diem. Dabei sollte eine Tagesdosis von 60 mg, vorzugsweise 30 mg Sennosid(en) vorteilhafterweise nicht überschritten werden. Um andererseits eine ausreichend abführende Wirkung zu erzielen, sollte die erfindungsgemäß Zusammensetzung aber in solchen Mengen verabreicht werden, daß die Tagesdosis an Sennosid(en) mindestens 5 mg, insbesondere mindestens 10 mg, vorzugsweise mindestens 15 mg, beträgt. Dabei kann es vorteilhaft sein, die Gesamttagesmenge auf mehrere Einzelgaben aufzuteilen, z. B. auf zwei bis drei Einzelgaben mit je 5 bis 25 g, insbesondere 5 bis 20 g, vorzugsweise 5 bis 10 g der Zusammensetzung pro Einzelgabe bzw. mit je 5 bis 20 mg, insbesondere 5 bis 10 mg Sennosid(en).

Die orale Einnahme sollte mit ausreichender Zufuhr von Wasser einhergehen, um einerseits eine effiziente Wirkung (= Quellung) zu gewährleisten und andererseits unerwünschte Nebenwirkungen (wie z. B. Verschluß der Speiseröhre) zu vermeiden; dies ist im allgemeinen dadurch sichergestellt, daß die orale Einnahme in Form einer wäßrigen Suspension der erfindungsgemäßen Pulverzusammensetzung erfolgt, da sich die erfindungsgemäße Zusammensetzung aufgrund der feinteiligen Pulverform ansonsten nicht einnehmen läßt. Im allgemeinen sollten bei der oralen Einnahme mindestens 40 ml Wasser pro 1 g der erfindungsgemäßen Zusammensetzung vorgesehen sein, wobei die erfindungsgemäße Zusammensetzung vor oraler Einnahme in dem Wasser in Suspension gebracht wird und anschließend in Form einer wäßrigen Suspension oral appliziert wird.

Die Herstellung der erfindungsgemäßen pulverförmigen Zusammensetzung kann in an sich bekannter Weise erfolgen. Die Herstellung erfolgt im allgemeinen nach den üblichen Herstellverfahren für Pulverzusammensetzungen. Dies kann beispielsweise dadurch geschehen, daß die einzelnen Bestandteile (gegebenenfalls nach Trocknung) zunächst fein vermahlen werden, wobei der Mahlungsgrad gezielt auf die gewünschte Granulometrie der Endzusammensetzung eingestellt werden kann, und anschließend eine innige homogene Mischung der einzelnen Bestandteile in den gewünschten Mengenverhältnissen hergestellt wird. Das Vermahlen der einzelnen Bestandteile kann aber alternativ auch erst im Zustand der Mischung erfolgen.

Weiter betrifft die vorliegende Erfindung die Verwendung der erfindungsgemäßen Zusammensetzung zur Herstellung eines Arzneimittels oder einer pharmazeutischen Zusammensetzung zur Förderung und/oder Erleichterung und/ oder Regulierung der Darmentleerung bzw. der Darmtätigkeit. Somit betrifft die vorliegende Erfindung die Verwendung der erfindungsgemäßen Zusammensetzung zur prophylaktischen und/oder kurativen (therapeutischen) Behandlung der vorgenannten Krankheitszuder Darmentleerung bzw. der Darmtätigkeit (z. B. zur Behandlung von Verstopfungen bzw. Obstipationen oder von anderen Erkrankungen, bei denen eine leichte Defäkation mit weichem Stuhl erwünscht ist, wie oben beschrieben), wobei die zuvor beschriebene erfindungsgemäße Zusammensetzung in therapeutisch wirksamen Mengen wird, im allgemeinen durch orale Applikation, vorzugsweise orale Zufuhr einer wäßrigen Suspension der pulverförmigen Zusammensetzung nach der vorliegenden Erfindung.

### Ausführungsbeispiele

### Beispiel 1

Eine erfindungsgemäße Zusammensetzung und zwei Vergleichsprodukte des Standes der Technik werden in eine wäßrige Suspension überführt. Zu diesem Zweck werden jeweils 6 g der Zusammensetzungen in je etwa 240 ml Leitungswasser eingerührt.

Die erfindungsgemäße Zusammensetzung ist ein feinteiliges Pulver (mehr als 70 Gew.-% der Teilchen mit mittleren Durchmessern im Bereich von 125 bis 250 µm), welches 52 Gew.-% Plantagosamen, 2,2 Gew.-% Plantagosamenschalen, 12,3 Gew.-% Tinnevelly-Sennafrüchte (entsprechend etwa 0,3 Gew.-% Sennosiden), 8,3 Gew.-% Guar-Gummi, 0,07 Gew.-% feinteilige Kieselsäure, 0,03 Gew.-% Maltodextrine und 25,1 Gew.-% weitere Inhaltsstoffe (natürliche Aroma-, Farb-, Geschmacks- und Süßungsstoffe) enthält.

Die erste Vergleichszusammensetzung ist eine Zusammensetzung aus 52 Gew.-% Plantagosamen, 2,2 Gew.-% Plantagosamenschalen, 10 bis 13,2 Gew.-% Tinnevelly-Sennafrüchten (entsprechend etwa 0,3 Gew.-% Sennosiden) und 32,6 bis 35,8 Gew.-% weiteren Inhaltsstoffen (Talkum, Gummi arabicum, Eisenoxide, Paraffin, Aromastoffe, Saccharose) enthält, aber frei von Guar-Gummi und Kieselsäure ist.

Die zweite Vergleichszusammensetzung ist ein Handelsprodukt auf Basis von indischen Flohsamenschalen (Psyllium), welches daneben weitere Inhaltsstoffe (unter anderem natürliche und künstliche Farb-, Geschmacks-, Aroma- und Süßungsstoffe, Zitronensäure, Eisenoxide, Calcium) enthält, aber frei von Sennosiden ist und auch kein Guar-Gummi und keine Kieselsäure enthält.

Nach Suspension der drei Zusammensetzungen in Wasser bleibt nur die erfindungsgemäße Zusammensetzung über mehr als eine Viertelstunde stabil, während die beiden Vergleichssuspensionen schon nach wenigen Minuten eine Phasentrennung zeigen (Sedimentation der Feststoffe). Die zweite Vergleichszusammensetzung, welche auch durch ihren faden Geschmack und das künstliche Aussehen nachteilig auffällt, geliert zudem nach einer Viertelstunde und bildet einen dicken, untrinkbaren Klumpen auf der Oberfläche, während die Suspension der erfindungsgemäßen Zusammensetzung auch noch nach einer Stunde trinkbar bleibt.

Auch in ihrer laxativen Wirkung unterscheiden sich die drei Zusammensetzungen signifikant: Während die zweite Vergleichszusammensetzung auf Basis von indischen Flohsamenschalen (Psyllium) nur eine mäßig abführende Wirkung zeigt, die in schwereren Fällen von Obstipationen nicht immer ausreichend ist, zeigt die erste Vergleichszusammensetzung eine deutlich bessere abführende Wirkung aufgrund der Kombination von physikalischer Wirkung der Plantagosamen/Plantagosamenschalen infolge von Quellung einerseits und pharmakologisch anregender Wirkung der Sennoside andererseits. Diese Kombinationswirkung wird bei der erfindungsgemäßen Zusammensetzung aufgrund der synergistischen Wirkweise des Guar-Gummis sogar noch gesteigert.

### Beispiel 2

Eine erfindungsgemäße Zusammensetzung wurde wie folgt hergestellt:

### Ausgangsmischung:

| **Bestandteil** | **g/Dosiseinheit** | **g/Charge** |
|---|---|---|
| Plantagosamen | 3,12 | 561.600 |
| Plantagosamenschalen | 0,13 | 23.400 |
| Tinnevelly-Sennafrüchte | 0,74 | 133.200 |
| Natürliche Farb- und Geschmacksstoffe | 1,5058 | 271.044 |
| Guar-Gummi | 0,5 | 90.000 |
| Aerosil^{®} | 0,0042 | 756 |
| | Σ 6,00 g | Σ 1.080.000 g |

### Sieben und Mischen:

Die einzelnen Bestandteile wurden - jeweils in feinstvermahlener Form - mittels Vakuum in einen Doppelkonusmischer (zweiseitig zugespitzter Mischer) mit 2.5001 Fassungsvolumen eingebracht. Klumpen bzw. größere Bestandteile wurden beim Beladen des Mischers über ein 3-mm-Sieb abgetrennt. Die Materialien wurden dann 30 Minuten lang bei einer Geschwindigkeit von 16 U/min vermischt. Anschließend wurde das homogenisierte, vermischte Produkt nach unten hin in zwei Polypropylen-Bigbags, die mit Polyethylen-Innensäcken ausgestattet waren, eingefüllt. Es resultierte eine erfindungsgemäße Mischung bzw. Zusammensetzung.

### Sachets-Füll- und Packprozeß:

Einer Maschine zur gleichzeitigen Befüllung von jeweils vier Sachets (Portionsbeuteln) wurde mittels Vakuum das zuvor hergestellte Pulverprodukt zugeführt. Die einzelnen Sachets wurden jeweils mit einer zuvor festgelegten Dosis von 6 g über einen Schraubendosierer beladen. Die Chargen-Nr. und das Verfallsdatum wurden auf der Falz der Sachets eingeprägt. Anschließend wurden die Sachets über Schweiß- bzw. Heizbakken verschweißt, so daß der Inhalt luft- und wasserdicht verpackt war. Die beladenen und verschweißten Sachets wurden dann paarweise insgesamt zu 20 Sachets aufgestapelt und zu einer Kartonier- bzw. Verpackungsmaschine transportiert, wo sie zusammen mit dem Beipackzettel in einen Karton verpackt wurden, der mit der jeweiligen Chargen-Nr. und dem Verfallsdatum bedruckt war. Anschließend wurden die Einheiten auf ihr korrektes Gewicht geprüft und gegebenenfalls abweichende Einheiten wurden verworfen. Eine festgelegte Anzahl der beladenen Kartons kann anschließend zu Großpackungen weiterverpackt werden.

## Patentansprüche

1. Pulverförmige Zusammensetzung, welche
(A) Plantagosamen (Plantaginis ovatae Semen) und/oder (A') Plantagosamenschalen; und
(B) mindestens eine anthranoide Verbindung, insbesondere mindestens ein Sennosid, vorzugsweise in Form von anthranoidhaltigen, insbesondere sennosidhaltigen Pflanzenteilen oder Pflanzenbestandteilen,
enthält,
**dadurch gekennzeichnet,**
**daß** die Zusammensetzung außerdem (C) mindestens ein Polygalactomannan oder dessen Derivat enthält.

2. Zusammensetzung nach Anspruch 1, **dadurch gekennzeichnet, daß** sie außerdem (D) mindestens eine Kieselsäure oder deren Derivat enthält, insbesondere eine hochdisperse, vorzugsweise pyrogene Kieselsäure, bevorzugt mit einem SiO₂-Gehalt von 95 % und mehr, insbesondere von 99 % und mehr, insbesondere wobei ein Teil der Kieselsäure oder ihres Derivates durch ein Maltodextrin ersetzt sein kann.

3. Zusammensetzung nach Anspruch 1 oder 2, **dadurch gekennzeichnet, daß** das Polygalactomannan oder seine Derivate ausgewählt sind aus der Gruppe von Guaran (Guar-Gummi, Cyamopsis-Gummi) und Guar-Derivaten, insbesondere partiell oder vollständig veresterten und/oder veretherten Guar-Derivaten, insbesondere Guarethem, wie Carboyxymethyl- und Hydroxyalkyl-Derivaten und kationisch modifizierten Produkten aus der Umsetzung von Guar-Mehl mit Monochloressigsäure, Ethylen- oder Propylenoxid und 2,3-Epoxypropyltrimethylammoniumchlorid in Gegenwart von Alkali.

4. Zusammensetzung nach einem oder mehreren der vorangehenden Ansprüche, **dadurch gekennzeichnet, daß** das Polygalactomannan oder sein Derivat in Form von Guar-Mehl und/oder Guar-Gummi zugesetzt ist.

5. Zusammensetzung nach einem oder mehreren der vorangehenden Ansprüche, **dadurch gekennzeichnet, daß** die Zusammensetzung ein Gemisch verschiedener anthranoider Verbindungen, insbesondere Sennoside, enthält.

6. Zusammensetzung nach einem oder mehreren der vorangehenden Ansprüche, **dadurch gekennzeichnet, daß** das Sennosid ein Hydroxyanthracenderivat, insbesondere ein 1,8-Dihydroxanthronderivat, ist und/oder daß das Sennosid ausgewählt ist aus der Gruppe der folgenden Verbindungen der allgemeinen Formel (I): 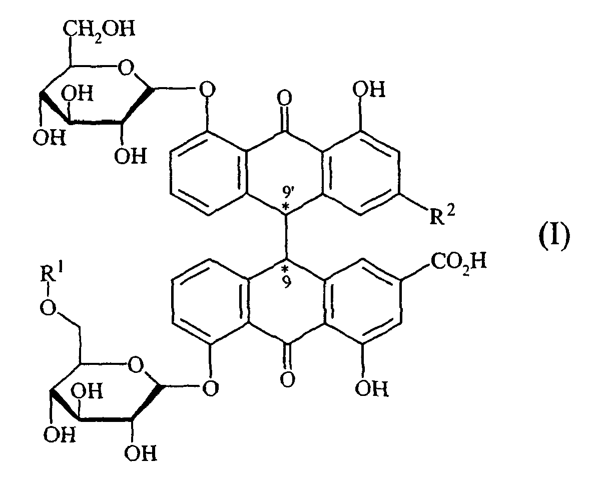 wobei in der allgemeinen Formel (I)
• der Rest R¹ Wasserstoff oder eine Gruppe -CO-CO₂H darstellt,
• der Rest R² eine Gruppe -CO₂H oder -CH₂OH darstellt, jedoch mit der Maßgabe, daß, wenn R¹ eine Gruppe -CO-CO₂H bezeichnet, R² eine Gruppe -CO₂H darstellt,
• die mit dem Zeichen "*" **gekennzeichnet**en Kohlenstoffatome in 9- und 9'-Position des Anthrongerüstes Chiralitätszentren darstellen,
sowie Mischungen und/oder Stereoisomeren, insbesondere Enantiomeren und/oder Diastereoisomeren, und/oder Derivaten der vorgenannten Verbindungen.

7. Zusammensetzung nach Anspruch 6, **dadurch gekennzeichnet, daß** das Sennosid ausgewählt ist aus der Gruppe der folgenden Verbindungen der allgemeinen Formel (I):
| Verbindung | R¹ | R² | 9-9' |
|---|---|---|---|
| (I A) | -H | -CO₂H | R*,R* (threo) |
| (I B) | -H | -CO₂H | R*,S* (erythro) |
| (I C) | -H | -CH₂OH | R*,R* (threo) |
| (I D) | -H | -CH₂OH | R*,S* (erythro) |
| (I E) | -CO-CO₂H | -CO₂H | R*,R* (threo) |
| (I F) | -CO-CO₂H | -CO₂H | R*,S* (erythro) |

8. Zusammensetzung nach einem oder mehreren der vorangehenden Ansprüche, **dadurch gekennzeichnet, daß** das oder die Sennosid(e) in Form von Früchten (Fructus), insbesondere Fruchthülsen, und/oder Fiederblättern (Folia), vorzugsweise Früchten, insbesondere Fruchthülsen, von Sennapflanzen zugesetzt sind, insbesondere der Alexandriner- und/oder der Tinnevelly-Sennapflanze, vorzugsweise der Tinnevelly-Sennapflanze, bevorzugt in getrockneter und zu feinem Pulver vermahlener Form.

9. Zusammensetzung nach Anspruch 8, **dadurch gekennzeichnet, daß** das oder die Sennoside in Form von Früchten und/oder Fiederblättern, vorzugsweise Früchten, insbesondere Fruchthülsen, der Tinnevelly-Sennapflanze (Senna angustifolia, Cassia angustifolia) zugesetzt sind.

10. Zusammensetzung nach einem oder mehreren der vorangehenden Ansprüche, **dadurch gekennzeichnet, daß** das oder die Sennoside, insbesondere die Sennapflanzenpartikel, von den Plantagosamen, insbesondere von den Schleimstoffen der Plantagosamen, zumindest teilweise oder vollständig umhüllt sind.

11. Zusammensetzung nach einem oder mehreren der vorangehenden Ansprüche, **gekennzeichnet durch** einen Gehalt an Plantagosamen von 30 bis 80 Gew.%, insbesondere 40 bis 70 Gew-%, vorzugsweise 45 bis 55 Gew.-%, und/oder **durch** einen Gehalt an Plantagosamenschalen von 0 bis 5 Gew.-%, insbesondere 1 bis 4 Gew.-%, vorzugsweise 1,5 bis 3 Gew.-%, und/oder **durch** einen Gehalt an Sennosid(en) von 0,05 bis 1,0 Gew.-%, insbesondere 0,08 bis 0,6 Gew.-%, vorzugsweise 0,1 bis 0,5 Gew.-%, und/oder **durch** einen Gehalt an Sennapflanze(n), insbesondere Sennafrüchten oder Sennafruchthülsen, von 5 bis 25 Gew.-%, insbesondere 7,5 bis 15 Gew.-%, vorzugsweise 10 bis 13 Gew.-%, und/ oder **durch** einen Gehalt an Polygalactomannan(en), insbesondere Guaran und/oder Guarderivaten von 5 bis 20 Gew.-%, insbesondere 7 bis 15 Gew.-%, vorzugsweise 7,5 bis 12 Gew.-%, besonders bevorzugt 8,0 bis 10 Gew.-%, und/oder einen Gehalt an Kieselsäure oder ihren Derivaten von 0 bis 5 Gew.-%, insbesondere 0,01 bis 2 Gew.-%, vorzugsweise 0,02 bis 1 Gew.-%, besonders bevorzugt 0,05 bis 0,1 Gew.%, wobei alle vorgenannten Gewichtsprozentangaben jeweils auf das Trockengewicht der Gesamtzusammensetzung bezogen sind.

12. Zusammensetzung nach einem der vorangehenden Ansprüche, **dadurch gekennzeichnet, daß** sie außerdem mindestens ein weiteres Additiv und/ oder Zusatzstoff enthält, insbesondere ausgewählt aus der Gruppe von Farbstoffen wie natürlichen oder naturidentischen Farbstoffen, Geschmacksmitteln, Geschmacksverstärkern und Aromastoffen, Stabilisatoren und (Co-)Stabilisatoren, Füllstoffen und Verarbeitungshilfsmitteln, sowie Mischungen der vorgenannten Verbindungen.

13. Zusammensetzung nach einem oder mehreren der vorangehenden Ansprüche, **gekennzeichnet durch** die folgende Formulierung, wobei die Mengenangaben jeweils auf das Trockengewicht der Gesamtzusammensetzung bezogen sind:
| | Gewichtsteile |
|---|---|
| • (A) Plantagosamen: | 30 bis 80, insbesondere 40 bis 70, vorzugsweise 45 bis 55 |
| • (A') Plantagosamenschalen: | 0 bis 5, insbesondere 1 bis 4, vorzugsweise 1,5 bis 3 |
| • (B) Tinnevelly-Sennafrüchte: | 5 bis 25, insbesondere 7,5 bis 15, vorzugsweise 10 bis 13 (entsprechend an Sennosiden 0,05 bis 1,0, insbesondere 0,08 bis 0,6, vorzugsweise 0,1 bis 0,5 Gewichtsteile) |
| • (C) Guar-Mehl und/oder Guar-Gummi: | 5 bis 20, insbesondere 7 bis 15, vorzugsweise 7,5 bis 12, besonders bevorzugt 8,0 bis 10 |
| • (D) Kieselsäure: | 0 bis 5, insbesondere 0,01 bis 2, vorzugsweise 0,02 bis 1, besonders bevorzugt 0,05 bis 1 |
| • (E) Geschmacks- und Farbstoffe: | 0 bis 60, insbesondere 20 bis 50, vorzugsweise 35 bis 45 |

14. Zusammensetzung nach einem oder mehreren der vorangehenden Ansprüche, **dadurch gekennzeichnet,**
**daß** die Zusammensetzung die folgende Korngrößenverteilung aufweist:
• mehr als 95 Gew.-%, insbesondere mehr als 99 Gew.-%, feiner als 500 µm;
• mehr als 70 Gew.-%, insbesondere mehr als 75 Gew.-%, feiner als 250 µm;
• mehr als 10 Gew.-%, insbesondere mehr als 15 Gew.-%, feiner als 125 µm,
wobei alle vorgenannten Gewichtsangaben jeweils auf das Trockengewicht der Gesamtzusammensetzung bezogen sind, und/oder daß der Hauptmassenanteil der Zusammensetzung, insbesondere mehr als 60 Gew.-%, insbesondere mehr als 70 Gew.-%, vorzugsweise mehr als 75 Gew.-%, Korngrößen im Bereich von 12S bis 250 µm aufweisen.

15. Zusammensetzung gemäß den Ansprüchen 1 bis 14 zur Verwendung als Laxativum (Abführmittel).

16. Verwendung der Zusammensetzung gemäß den Ansprüchen 1 bis 14 zur Herstellung eines Arzneimittels oder einer pharmazeutischen Zusammensetzung zur Förderung und/oder Erleichterung und/oder Regulierung der Darmentleerung und/oder der Darmtätigkeit.

17. Verwendung nach Anspruch 16 zur prophylaktischen oder kurativen Behandlung von Erkrankungen, bei denen eine leichte Defäkation mit weichem Stuhl erwünscht ist, insbesondere Hämorrhoidalerkrankungen, Zuständen nach rektal-analen operativen Eingriffen und vor und nach operativen Eingriffen des Bauchraums sowie Obstipationen, oder zur Erleichterung der Darmpassage und Defäkation, insbesondere als Linderung der Nebenwirkungen, bei Therapie mit Medikamenten, die Obstipation verursachen.

18. Zusammensetzung nach Anspruch 15 oder Verwendung nach Anspruch 17, **dadurch gekennzeichnet, daß** die Zusammensetzung oral eingenommen wird, vorzugsweise als wäßrige Suspension, insbesondere in Tagesdosen von insgesamt 1 bis 50 g, insbesondere 1 bis 20 g, vorzugsweise 5 bis 10 g, der Zusammensetzung.

## Claims

1. A composition in powder form which comprises
(A) plantago seeds (plantaginis ovatae semen) and/or (A') plantago seed husks; and
(B) at least one anthranoid compound, in particular at least one sennoside, preferably in the form of anthranoid-containing, in particular sennoside-containing plant parts or plant constituents,
**characterized in that**
the composition additionally comprises (C) at least one polygalactomannan or derivative thereof.

2. Composition according to Claim 1, **characterized in that** it additionally comprises (D) at least one silica or derivative thereof, in particular a colloidal, preferably pyrogenic silica, preferably having an SiO₂ content of 95% and more, in particular of 99% and more, in particular where part of the silica or its derivative may be replaced by a maltodextrin.

3. Composition according to Claim 1 or 2, **characterized in that** the polygalactomannan or its derivatives are selected from the group of guaran (guar gum, cyamopsis gum) and guar derivatives, in particular partly or completely esterified and/or etherified guar derivatives, in particular guar ethers such as carboxymethyl and hydroxyalkyl derivatives and cationically modified products from the reaction of guar flour with monochloroacetic acid, ethylene oxide or propylene oxide and 2,3-epoxypropyltrimethylammonium chloride in the presence of alkali.

4. Composition according to one or more of the preceding claims, **characterized in that** the polygalactomannan or its derivative is added in the form of guar flour and/or guar gum.

5. Composition according to one or more of the preceding claims, **characterized in that** the composition comprises a mixture of different anthranoid compounds, especially sennosides.

6. Composition according to one or more of the preceding claims, **characterized in that** the sennoside is a hydroxyanthracene derivative, in particular a 1,8-dihydroxanthrone derivative, and/or **in that** the sennoside is selected from the group of the following compounds of the general formula (I): 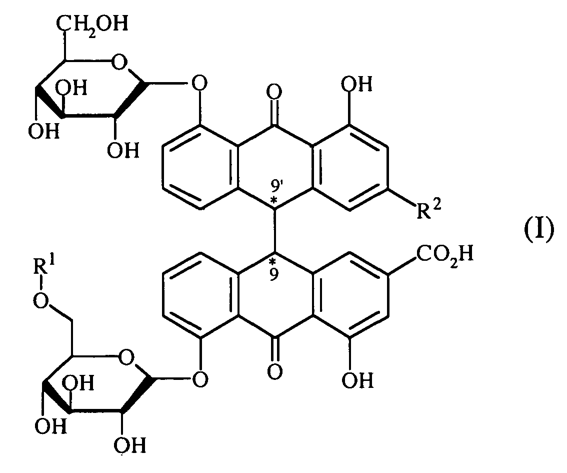 where in the general formula (I)
• the radical R¹ represents hydrogen or a -CO-CO₂H group,
• the radical R² represents a -CO₂H or -CH₂OH group, but with the proviso that R² represents a -CO₂H group when R¹ designates a -CO-CO₂H group,
• the carbon atoms identified by the sign "*" in positions 9 and 9' of the anthrone structure represent chirality centres,
and mixtures and/or stereoisomers, in particular enantiomers and/or diastereoisomers, and/or derivatives of the aforementioned compounds.

7. Composition according to Claim 6, **characterized in that** the sennoside is selected from the group of the following compounds of the general formula (I):
| Compound | R¹ | R² | 9-9' |
|---|---|---|---|
| (I A) | -H | -CO₂H | R*,R* (threo) |
| (I B) | -H | -CO₂H | R*,S* (erythro) |
| (I C) | -H | -CO₂OH | R*,R* (threo) |
| (I D) | -H | -CO₂OH | R*,S* (erythro) |
| (I E) | -CO-CO₂H | -CO₂H | R*,R* (threo) |
| (I F) | -CO-CO₂H | -CO₂H | R*,S* (erythro) |

8. Composition according to one or more of the preceding claims, **characterized in that** the sennoside(s) are added in the form of fruits (fructus), in particular fruit husks, and/or pinnate leaves (folia), preferably fruits, in particular fruit husks, of senna plants, in particular of the Alexandria and/or of the Tinnevelly senna plants, preferably of the Tinnevelly senna plant, preferably in a form which is dried and ground to a fine powder.

9. Composition according to Claim 8, **characterized in that** the sennoside(s) are added in the form of fruits and/or pinnate leaves, preferably fruits, in particular fruit husks, of the Tinnevelly senna plant (Senna angustifolia, Cassia angustifolia).

10. Composition according to one or more of the preceding claims, **characterized in that** the sennoside(s), in particular the senna plant particles, are at least partly or completely encased by the plantago seeds, in particular by the gummy substances of the plantago seeds.

11. Composition according to one or more of the preceding claims, **characterized by** a plantago seed content of from 30 to 80% by weight, in particular 40 to 70% by weight, preferably 45 to 55% by weight, and/or by a plantago seed husk content of from 0 to 5% by weight, in particular 1 to 4% by weight, preferably 1.5 to 3% by weight, and/or by a sennoside(s) content of from 0.05 to 1.0% by weight, in particular 0.08 to 0.6% by weight, preferably 0.1 to 0.5% by weight, and/or by a content of senna plant(s), in particular senna fruits or senna fruit husks, of from 5 to 25% by weight, in particular 7.5 to 15% by weight, preferably 10 to 13% by weight, and/or by a content of polygalactomannan(s), in particular guaran and/or guar derivatives, of from 5 to 20% by weight, in particular 7 to 15% by weight, preferably 7.5 to 12% by weight, particularly preferably 8.0 to 10% by weight, and/or a content of silica or its derivatives of from 0 to 5% by weight, in particular 0.01 to 2% by weight, preferably 0.02 to 1% by weight, particularly preferably 0.05 to 0.1 % by weight, where all the aforementioned percentage by weight data are in each case based on the dry weight of the complete composition.

12. Composition according to any of the preceding claims, **characterized in that** it additionally comprises at least one further additive and/or added substance, in particular selected from the group of colorants such as natural or nature-identical colorants, flavourings, flavour enhancers and aromatizing agents, stabilizers and (co)stabilizers, fillers and processing aids, and mixtures of the aforementioned compounds.

13. Composition according to one or more of the preceding claims, **characterized by** the following formulation, where the quantitative data are in each case based on the dry weight of the complete composition:
| | Parts by weight |
|---|---|
| • (A) Plantago seeds: | 30 to 80, in particular 40 to 70, preferably 45 to 55 |
| • (A') Plantago seed husks: | 0 to 5, in particular 1 to 4, preferably 1.5 to 3 |
| • (B) Tinnevelly senna fruits: | 5 to 25, in particular 7.5 to 15, preferably 10 to 13 (equivalent to sennosides 0.05 to 1.0, in particular 0.08 to 0.6, preferably 0.1 to 0.5, parts by weight) |
| • (C) Guar flour and/or guar gum: | 5 to 20, in particular 7 to 15, preferably 7.5 to 12, particularly preferably 8.0 to 10 |
| • (D) Silica: | 0 to 5, in particular 0.01 to 2, preferably 0.02 to 1, particularly preferably 0.05 to 1 |
| • (E) Flavourings and colorants: | 0 to 60, in particular 20 to 50, preferably 35 to 45 |

14. Composition according to one or more of the preceding claims, **characterized in that** the composition exhibits the following particle size distribution:
• more than 95% by weight, in particular more than 99% by weight, finer than 500 µm,
• more than 70% by weight, in particular more than 75% by weight, finer than 250 µm,
• more than 10% by weight, in particular more than 15% by weight, finer than 125 µm,
where all the aforementioned weight data are in each case based on the dry weight of the complete composition, and/or **in that** the chief mass fraction of the composition exhibit in particular more than 60% by weight, in particular more than 70% by weight, preferably more than 75% by weight, particle sizes in the range from 125 to 250 µm.

15. Composition according to Claims 1 to 14 for use as laxative (cathartic).

16. Use of the composition according to Claims 1 to 14 for manufacturing a medicament or a pharmaceutical composition for promoting and/or facilitating and/or regulating bowel evacuation and/or bowel activity.

17. Use according to Claim 16 for the prophylactic or curative treatment of disorders in which easy defaecation with soft stool is desired, in particular haemorrhoidal disorders, conditions following rectal/anal surgical procedures and before and after surgical procedures on the abdominal cavity, and constipations, or for facilitating passage through the bowel and defaecation, in particular for alleviating the side effects on therapy with medicaments which cause constipation.

18. Composition according to Claim 15 or use according to Claim 17, **characterized in that** the composition is taken orally, preferably as aqueous suspension, in particular in daily doses totally from 1 to 50 g, in particular 1 to 20 g, preferably 5 to 10 g, of the composition.

## Revendications

1. Composition pulvérulente qui contient
(A) des graines de psyllium des Indes (Plantaginis ovatae semen) et/ou (A') des téguments de graines de Psyllium des Indes ; et
(B) au moins un composé anthranoïde, en particulier au moins un sennoside, de préférence sous forme de parties de plantes ou de composants de plantes contenant un anthranoïde, en particulier contenant un sennoside,
**caractérisée en ce que**
la composition en outre contient (C) au moins un polygalactomannane ou un dérivé de celui-ci.

2. Composition selon la revendication 1, **caractérisée en ce qu'**en outre elle contient (D) au moins un acide silicique ou un dérivé de celui-ci, en particulier un acide silicique hautement dispersé, de préférence pyrogéné, de préférence ayant une teneur en SiO₂ de 95 % ou plus, en particulier de 99 % ou plus, en particulier une partie de l'acide silicique ou de son dérivé pouvant être remplacée par une maltodextrine.

3. Composition selon la revendication 1 ou 2, **caractérisée en ce que** le polygalactomannane ou ses dérivés sont choisis dans le groupe du guaranne ou de ses dérivés (gomme guar, gomme de cyamopsis) et des dérivés de guar, en particulier des dérivés de guar partiellement ou totalement estérifiés et/ou éthérifiés, en particulier des éthers de guar, tels que des dérivés carboxyméthyliques et hydroxyalkyliques et des produits à modification cationique provenant de la réaction de farine de guar avec l'acide monochloracétique, l'oxyde d'éthylène ou de propylène et le chlorure de 2,3-époxypropyltriméthylammonium en présence d'alcali.

4. Composition selon une ou plusieurs des revendications précédentes, **caractérisée en ce que** le polygalactomannane ou son dérivé est ajouté sous forme de farine de guar et/ou de gomme guar.

5. Composition selon une ou plusieurs des revendications précédentes, **caractérisée en ce que** la composition est un mélange de divers composés anthranoïdes, en particulier de sennosides.

6. Composition selon une ou plusieurs des revendications précédentes, **caractérisée en ce que** le sennoside est un dérivé d'hydroxyanthracène, en particulier un dérivé de 1,8-dihydroxanthrone et/ou le sennoside est choisi dans le groupe des composés suivants de formule générale (I) : 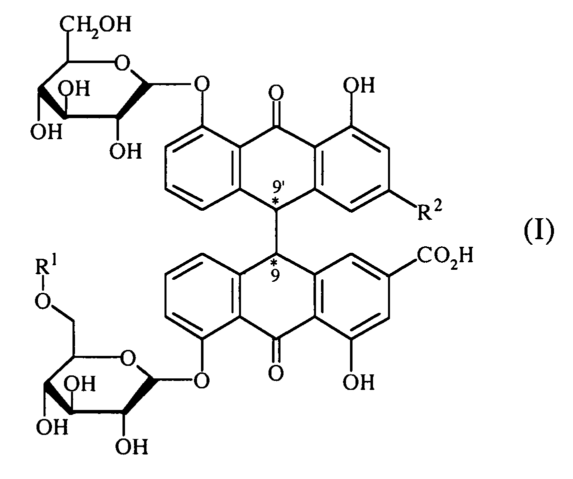 dans la formule générale (I)
• le radical R¹ représentant un atome d'hydrogène ou un groupe de formule -CO-CO₂H,
• le radical R² représentant un groupe -CO₂H ou -CH₂OH, mais avec la condition que lorsque R¹ désigne un groupe -CO-CO₂H, R² représente un groupe -CO₂H,
• les atomes de carbone **caractérisés par** le signe "*" en positions 9 et 9' du squelette anthrone représentant des centres de chiralité,
ainsi que des mélanges et/ou stéréoisomères, en particulier des énantiomères et/ou diastéréoisomères, et/ou des dérivés des composés précités.

7. Composition selon la revendication 6, **caractérisée en ce que** le sennoside est choisi dans le groupe des composés suivants de formule générale (I) :
| Composé | R¹ | R² | 9-9' |
|---|---|---|---|
| (IA) | -H | -CO₂H | R*,R* (thréo) |
| (I B) | -H | -CO₂H | R*,S* (érythro) |
| (I C) | -H | -CH₂OH | R*,R* (thréo) |
| (I D) | -H | -CH₂OH | R*,S* (érythro) |
| (I E) | -CO-CO₂H | -CO₂H | R*,R* (thréo) |
| (I F) | -CO-CO₂H | -CO₂H | R*,S* (érythro) |

8. Composition selon une ou plusieurs des revendications précédentes, **caractérisée en ce que** le ou les sennoside(s) est(sont) ajouté(s) sous formes de fruits (fructus), en particulier de gousses de fruits, et/ou de pennes (folia), de préférence de fruits, en particulier de gousses de fruits, de sénés, en particulier du séné de Tinnevelly et/ou du séné d'Alexandrie, de préférence du séné de Tinnevelly, de préférence sous forme séchée et broyée en fine poudre.

9. Composition selon la revendication 8, **caractérisée en ce que** le ou les sennosides sont ajoutés sous forme de fruits et/ou de pennes, de préférence de fruits, en particulier de gousses de fruits, du séné de Tinnevelly (*Senna angustifolia, Cassia angustifolia*).

10. Composition selon une ou plusieurs des revendications précédentes, **caractérisée en ce que** le ou les sennosides, en particulier les particules de séné, sont au moins partiellement ou totalement enveloppés par les graines de Psyllium des Indes, en particulier par les mucilages des graines de Psyllium des Indes.

11. Composition selon une ou plusieurs des revendications précédentes, **caractérisée par** une teneur en graines de Psyllium des Indes de 30 à 80 % en poids, en particulier de 40 à 70 % en poids, de préférence de 45 à 55 % en poids, et/ou par une teneur en téguments de graines de Psyllium des Indes de 0 à 5 % en poids, en particulier de 1 à 4 % en poids, de préférence de 1,5 à 3 % en poids, et/ou par une teneur en sennoside(s) de 0,05 à 1,0 % en poids, en particulier de 0,08 à 0,6 % en poids, de préférence de 0,1 à 0,5 % en poids, et/ou par une teneur en séné(s), en particulier en fruits de séné ou en gousses de séné, de 5 à 25 % en poids, en particulier de 7,5 à 15 % en poids, de préférence de 10 à 13 % en poids, et/ou par une teneur en polygalactomannane(s), en particulier guaranne et/ou dérivés de guaranne, de 5 à 20 % en poids, en particulier de 7 à 15 % en poids, de préférence de 7,5 à 12 % en poids, de façon particulièrement préférée de 8,0 à 10 % en poids, et/ou une teneur en acide silique ou ses dérivés de 0 à 5 % en poids, en particulier de 0,01 à 2 % en poids, de préférence de 0,02 à 1 % en poids, de façon particulièrement préférée de 0,05 à 0,1 % en poids, toutes les données en pourcentage en poids précitées étant rapportées chacune au poids sec de la composition totale.

12. Composition selon une ou plusieurs des revendications précédentes, **caractérisée en ce qu'**en outre elle contient au moins un autre additif et/ou adjuvant, en particulier choisi dans le groupe des colorants tels que des colorants naturels ou identiques aux colorants naturels, agents de sapidité, exhausteurs de goût et arômes, stabilisants et (co)-stabilisants, excipients et adjuvants de fabrication, ainsi que des mélanges des composés précités.

13. Composition selon une ou plusieurs des revendications précédentes, **caractérisée par** la formulation suivante, les données quantitatives étant chacune par rapport au poids sec de la composition totale :
| | Parties en poids |
|---|---|
| • (A) Graines de Psyllium des Indes: | 30 à 80, en particulier 40 à 70, de préférence 45 à 55 |
| • (A') Téguments de graines de Psyllium des Indes: | 0 à 5, en particulier 1 à 4, de préférence 1,5 à 3 |
| • (B) Fruits de séné de Tinnevelly : | 5 à 25, en particulier 7,5 à 15, de préférence 10 à 13 (correspondant à sennosides 0,05 à 1,0, en particulier 0,08 à 0,6, de préférence 0,1 à 0,5 parties en poids) |
| • (C) Farine de guar et/ou gomme guar: | 5 à 20, en particulier 7 à 15, de préférence 7,5 à 12, de façon particulièment préférée 8,0 à 10 |
| • (D) Acide silicique : | 0 à 5, en particulier 0,01 à 2, de préférence 0,02 à 1, de façon particulièrement préférée 0,05 à 1 |
| • (E) Agents de sapidité et colorants : | 0 à 60, en particulier 20 à 50, de préférence 35 à 45. |

14. Composition selon une ou plusieurs des revendications précédentes, **caractérisée en ce que** la composition présente la distribution granulométrique suivante :
• plus de 95 % en poids, en particulier, plus de 99 % en poids de particules de taille inférieure à 500 µm ;
• plus de 70 % en poids, en particulier, plus de 75 % en poids, de particules de taille inférieure à 250 µm ;
• plus de 10 % en poids, en particulier plus de 15 % en poids, de particules de taille inférieure à 125 µm,
toutes les données pondérales précitées étant chacune par rapport au poids sec de la composition, et/ou
**en ce que** la proportion en masse principale, en particulier plus de 60 % en poids, en particulier plus de 70 % en poids, de préférence plus de 75 % de la composition ont des tailles de particule dans la plage de 125 à 250 µm.

15. Composition selon les revendications 1 à 14, pour l'utilisation en tant que laxatif (purge).

16. Utilisation de la composition selon les revendications 1 à 14, pour la fabrication d'un médicament ou d'une composition pharmaceutique pour encourager et/ou faciliter et/ou régulariser l'évacuation intestinale et/ou le péristaltisme intestinal.

17. Utilisation selon la revendication 16, pour le traitement prophylactique ou curatif d'affections dans lesquelles est souhaitée une défécation aisée avec des selles molles, en particulier d'affections hémorroïdales, d'états après des interventions opératoires rectales-anales et avant des interventions opératoires de la cavité abdominale ainsi que de constipations, ou pour faciliter le transit intestinal et la défécation, en particulier comme soulagement des effets secondaires dans la thérapie par des médicaments qui provoquent la constipation

18. Composition selon la revendication 15 ou utilisation selon la revendication 17, **caractérisée en ce que** la composition est prise par voie orale, de préférence sous forme de suspension aqueuse, en particulier à des doses journalières de 1 à 50 g, en particulier de 1 à 20 g, de préférence de 5 à 10 g, de la composition.
